# EUROPEAN PATENT APPLICATION

(11) **EP 4 213 160 A1**
(43) Date of publication of application: **19.07.2023**
(21) Application number: 21858668.3
(22) Date of filing: 23.08.2021
(51) Int. Cl.: G16H 50/80, G16H 10/20, G16H 50/20, G16H 10/60, G16H 50/70, G16H 50/30, G16H 40/63, G16H 80/00, G16H 15/00

(54) **PERSONAL MEDICAL RECORD SYSTEM AND METHOD RELATED TO NONCONTACT SCREENING TREATMENT FOR LARGE-SCALE INFECTIOUS DISEASE**

(30) Priority: 21.08.2020 KR 20200105012
(71) Applicant: Park, Yong Nam, Gyeonggi-do 10915 (KR)
(72) Inventor: Park, Yong Nam, Gyeonggi-do 10915 (KR)
(74) Representative: Cabinet Chaillot
(86) International application number: PCT/KR2021/011224
(87) International publication number: WO 2022/039581

(57) **Abstract**

The present technology provides a personal medical record system and method related to noncontact screening treatment for a large-scale infectious disease. On the basis of the present specific embodiment, the filling out of an electronic questionnaire including a pre-epidemiological survey for large-scale infectious disease, a diagnosis reservation, and diagnosis can be carried out in an noncontact manner on a cloud-based open mobile platform that is open source by utilizing cloud computing, a diagnosis request text message and an electronic questionnaire are transmitted to terminal information of a person suspected to have come in contact with a confirmed case, so that the mass infection can be quickly prevented, a prescription service for transmitting a medical prescription and payment information to an examinee terminal in an noncontact manner can be provided, a diagnostic certification service for authenticating a diagnosis result about a large-scale infectious disease on the basis of a diagnosis result of a medical institution terminal can be provided, and arrival information about a corresponding store, having been acquired by a tagging operation of the examinee terminal, is collected and managed in an entrance terminal placed at a predetermined position of a visited store, so that an arrival certification service can be provided.

## Description

### TECHNICAL FIELD

The present invention relates to a personal medical record system and method related to noncontact screening treatment for a large-scale infectious disease including a preliminary epidemiological investigation at an appointment stage, and more specifically, to a technique that allows more efficient testing and collective quarantine, in which as a screening treatment appointment is made and screening treatment is conducted for a large-scale infectious disease in a noncontact manner, and the screening treatment appointment and screening treatment are progressed in real-time without an electronic medical record system like in a conventional method, it is possible to prevent large-scale mass cross-infection highly likely to occur in the registration process at a screening treatment site, reduce the time of diagnosing a large-scale infectious disease to the minimum, and properly conduct a preliminary epidemiological investigation on related information needed at an appointment stage without fail.

### BACKGROUND ART

Due to the problem of patients suspected of being infected with an infectious disease caused by new viruses of a high mortality rate such as swine flu, SARS, MERS, and COVID-19, which are pervasive across the world recently, it is necessary to find infected patients as soon as possible and immediately isolate and treat them intensively by means of screening treatment including medical interview, test, confirmation, and the like systematically performed for a corresponding infectious disease at the national level.

The screening treatment is not a medical activity in an existing space of a legal medical institution such as a clinic or a hospital, but refers to a medical activity such as diagnosis, testing, and the like conducted by medical personnel legally reported to and certified by medical authorities in relation to screening treatment activities in order to confirm and screen infections for patients suspected of being infected with a corresponding infectious disease in an environment with very little risk of infection, such as outdoors, mobile negative pressure spaces, positive pressure booths, rooftops, or the like.

However, before the screening treatment is conducted for the people suspected of being infected with a large-scale infectious disease, those who wish to receive screening treatment need to fill out in detail and prepare a corresponding preliminary epidemiological investigation form including some tens of items, such as personal information (name, address, resident registration number (social security number or passport number in the case of foreigners), contact information, vehicle number, etc.), symptom details, detailed information related to the preliminary epidemiological investigation (including information on contact with an existing specific confirmed patient, overlapped movement paths, etc.), and the like. However, as most of the screening treatment applicants fill out paper-type treatment applications provided at a field screening clinic, there is rather a high risk of cross-infection due to the tools themselves related to the treatment, such as papers, paper stands, or the like provided at the field. In addition, as the waiting time at the field screening treatment is extended, there is a high risk of cross-infection among the people standing in line, and as the disease spreads by droplet infection through questions and answers in the related processes (prior guidance, registration, interview, etc.), the risk of side effects such as contamination of the screening treatment field space, cross-infection, and the like is very high.

Moreover, even in the digital age, operation of screening clinics is carried out through a manual work (preparation of medical questionnaires, epidemiological investigation reports, etc.) and face-to-face consultation since online appointments and automatic systems are not equipped unlike university hospitals, and thus inconvenience of residents has been aggravated due to overwork of persons in charge and unnecessary visits.

In addition, there is no epidemiological investigation system (including information on contact with specific confirmed patients, overlapped movement paths, etc.) at a field screening clinic previously, and thus an examiner, who is a doctor, should determine whether or not to take a test and whether or not to enforce self-quarantine and guide a self-quarantine period on the spot only on the basis of the statements of an examinee in complete ignorance of previously identified epidemiological investigation information on an examinee, and therefore, it is very difficult to make a right decision and guidance, and as a wrong decision and guidance on whether or not to have a test is made due to the problem, there occurs a secondary problem of making it difficult to effectively prevent spread of large-scale infection and excessively making unnecessary tests.

In addition, a corresponding screening clinic needs to organize all the information recorded in a corresponding form into computerized information and report the information to the infectious disease control health authorities when an infectious disease occurs as a legal obligation or the like of reporting corresponding contents to the health authorities, and when the information is recorded in a paper form, there is a burdensome problem of requiring a lot of effort in computerizing the information additionally.

Therefore, the inventors of the present invention propose a method of simultaneously promoting even a noncontact mobile appointment service in order to improve inefficiency of the working process through visit to a public health center, block sources of infection generated in a social distancing and treatment process, and provide a noncontact examination environment.

Accordingly, when a integrated information system is constructed for the screening clinics, applicants of a test may make an appointment (prepare a medical questionnaire, epidemiological investigation report, and the like) using a mobile device, visit a clinic, have an interview with a doctor, take a sample, and receive a result later through a text message. In addition, arrival and registration (recognition using a QR code reader), medical interview with a doctor, collection of samples (output a barcode label), request for a test, notification of a test result, and the like may be performed at the clinic at the same time.

In addition, as contact information of a contact suspect is recorded when an applicant makes an appointment and a push message is transmitted to the contact suspect as soon as the applicant is confirmed to be infected, a large-scale infectious disease of the contact suspect may be diagnosed in an early stage, and spread of the large-scale infectious disease can be prevented.

### DISCLOSURE OF INVENTION

### TECHNICAL PROBLEM

An object of the present invention is to provide a personal medical record system and method related to noncontact screening treatment for a large-scale infectious disease, which can prevent a large-scale mass infection in advance by face-to-face treatment registration and medical treatment as preparation of an electronic medical questionnaire including a preliminary epidemiological investigation report for the large-scale infectious disease, a screening treatment appointment, and screening treatment are performed in a noncontact manner using a cloud-based open mobile platform having an open source by utilizing cloud computing.

Another object of the present invention is to provide a personal medical record system and method related to noncontact screening treatment for a large-scale infectious disease, which can prevent further spread of a large-scale infectious disease in advance since a push message is transmitted to a contact suspect as soon as an examinee is diagnosed as being infected as terminal information of a contact suspect is recorded at the appointment stage.

The objects of the present invention are not limited to the objects mentioned above, and other unmentioned objects and advantages of the present invention may be understood by the following description and will be more clearly known by the embodiments of the present invention. Furthermore, it will be easily understood that the objects and advantages of the present invention may be realized by the means indicated in the claims and combinations thereof.

### TECHNICAL SOLUTION

According to one aspect of the present invention, there is provided a personal medical record system related to noncontact screening treatment for a large-scale infectious disease, the system comprising:
a management server having a cloud-based mobile platform constructed therein to preside and mediate preparation of an electronic medical questionnaire including a preliminary epidemiological investigation for the large-scale infectious disease; and
an examinee terminal operating in association with the management server and equipped with an app (including a PC or a mobile web browser site) for providing an electronic medical questionnaire preparation service including a preliminary epidemiological investigation for the large-scale infectious disease to download an electronic medical questionnaire input window by executing the app and transmit the prepared electronic medical questionnaire to the management server by pressing an input confirmation key.

Preferably, the management server performs the electronic medical questionnaire preparation service in a noncontact manner by
transmitting an electronic medical questionnaire, which contains a plurality of forms of consent to information sharing and inquiries about personal identification information of the examinee, clinical symptoms, terminal information of a contact suspect, and the preliminary epidemiological investigation, to the subject terminal, and
transmitting the received prepared electronic medical questionnaire to a medical institution terminal by pressing the input confirmation key of the examinee terminal.

Preferably, the consent form of the electronic medical questionnaire may include
at least one among consent to collection of personal health record (PHR), consent to sharing of information among participating medical institutions, consent to sharing of personal information for research purposes, and consent to omitting input of personal identification information in the case of an anonymous diagnosis.

Preferably, as an anonymous test item for preventing exposure of the personal identification information is added to the electronic medical questionnaire in the electronic medical questionnaire input window, the management server may be provided to
perform identity authentication using a communication subscription number of the examinee for receiving a notification of a test result, and prepare an electronic medical questionnaire omitting input of a personal identification information item when the identity authentication is successful.

Preferably, the management server is provided to
provide a diagnosis appointment service for the examinee according to an isolation stage received from the medical institution terminal, and
provide the diagnosis appointment service in a noncontact manner by mapping a plurality of screening clinics available for making an appointment to map data on the basis of an isolation measure of the medical institution terminal set based on the clinical symptoms and preliminary epidemiological investigation of the electronic medical questionnaire, and transmitting the screening clinics to the examinee terminal,
transmitting an input window of diagnosis appointment information, including an appointment date of a screening clinic selected among displayed screening clinics, a time slot, a diagnosis method of noncontact diagnosis or drive-through diagnosis, and a license plate number when the drive-through diagnosis is selected, to the examinee terminal, and
generating a diagnosis appointment number including appointment status of the selected screening clinic on the basis of the received appointment information when corresponding items of the diagnosis appointment information are input and the input confirmation key is pressed and transmitting the diagnosis appointment number to the examinee terminal.

Preferably, a plurality of selected screening clinics selected by the management server may be
one of a screening clinic with a copay or a screening clinic without a copay among the plurality of screening clinics according to contents of the electronic medical questionnaire filled out in advance.

Preferably, the management server is provided to
provide a diagnostic test service for a large-scale infectious disease when the examinee arrives at the selected screening clinic, and
the management server may be provided to perform an arrival authentication of the examinee at the selected screening clinic by inputting the diagnosis appointment number through an authentication input unit provided at a predetermined location of the selected screening clinic or based on a barcode or license plate number acquired through a camera, generate a diagnosis registration number matching the diagnosis appointment number when the authentication is successful,
generate as needed a labeling barcode, in which personal identification information matching the generated diagnosis registration number is encrypted and stored, after a doctor orders a test, and
perform identity authentication for the generated labeling barcode, attach the labeling barcode to a diagnosis kit of the examinee, and transmit the diagnosis kit to the medical institution terminal when the authentication is successful.

Preferably, the management server may be provided to
provide a prescription service of transmitting at least one among a diagnosis result of the medical institution terminal, a medical prescription, and payment information to at least one among the examinee terminal, a drug store, a courier server, and a payment server in a noncontact manner.

Preferably, the management server may be provided to
provide a diagnosis certification service of certificating a diagnosis result about a large-scale infectious disease by storing and managing diagnosis results of the medical institution terminal by personal identification information and transmitting the diagnosis result to the examinee terminal in a noncontact manner in real-time.

Preferably, the management server may be provided to
transmit a diagnosis request text message and an electronic medical questionnaire generated based on the diagnosis result to the terminal of the contact suspect recorded in the electronic medical questionnaire of a confirmed examinee, and the diagnosis request text message and the electronic medical questionnaire are transmitted in the form of a push message.

Preferably, the management server may be provided to
provide a visit certification service by collecting and managing information on a visit to a corresponding store acquired through a tagging operation of the examinee terminal on an entrance terminal provided at a predetermined location of the visited store, and transmitting information on the visit to the corresponding store to the examinee terminal in response to a request of the examinee terminal.

According to another aspect of the present invention, there is provided a personal medical record method related to noncontact screening treatment for a large-scale infectious disease,
in a noncontact screening treatment method performed by a noncontact screening treatment appointment system including a management server having a cloud-based mobile platform and an examinee terminal connected based on a web, the method comprising the steps of:
mounting an app (including a PC or mobile web program) of the management server for providing an electronic medical questionnaire preparation service including a preliminary epidemiological investigation for the large-scale infectious disease on the examinee terminal, and executing the mounted app;
transmitting, by the management server, an electronic medical questionnaire input window, containing a plurality of forms of consent to information sharing and inquiries about personal identification information of an examinee, clinical symptoms, terminal information of a contact suspect, and the preliminary epidemiological investigation, to the examinee terminal; and
transmitting, by the management server after preparing the electronic medical questionnaire, the received electronic medical questionnaire to the medical institution terminal by pressing an input confirmation key of the examinee terminal.

Preferably, the personal medical record method related to noncontact screening treatment for a large-scale infectious disease is provided to
provide a diagnosis appointment service for the examinee according to an isolation stage received from the medical institution terminal, by the management server, and may further comprise the steps of:
mapping a plurality of screening clinics available for making an appointment to map data on the basis of an isolation stage set based on the clinical symptoms and preliminary epidemiological investigation received from the medical institution terminal, and transmitting the screening clinics to the examinee terminal, by the management server;
transmitting an input window of appointment information, which includes an available date of a screening clinic selected among displayed screening clinics, a time slot, a diagnosis method of noncontact diagnosis or drive-through diagnosis, and a license plate number when the drive-through diagnosis is selected, to the examinee terminal; and
generating a diagnosis appointment number including appointment status of the selected screening clinic on the basis of the received appointment information when each item of the appointment information is input and the input confirmation key of the examinee terminal is pressed and transmitting the diagnosis appointment number to the examinee terminal.

Preferably, the personal medical record method related to noncontact screening treatment for a large-scale infectious disease
conducts diagnosis of a large-scale infectious disease in a noncontact manner when the examinee arrives at the selected screening clinic, by the management server, and may be provide to
generate a diagnosis registration number matching the diagnosis appointment number at the time of arrival authentication of the examinee on the basis of input of the diagnosis appointment number of the authentication input unit provided at a predetermined location of the selected screening clinic, or a barcode or a license plate number acquired through a camera,
generate a labeling barcode, in which personal identification information matching the generated diagnosis registration number is encrypted and stored, after a doctor orders a test, and
attach the labeling barcode to a diagnosis kit of the examinee, and transmit the diagnosis kit to the medical institution terminal when identity authentication for the generated labeling barcode is successful.

Preferably, the personal medical record method related to noncontact screening treatment for a large-scale infectious disease may be provided to
transmit, by the management server, a diagnosis request text message and an electronic medical questionnaire, which is generated when the examinee is confirmed to be infected as a result of the diagnosis, to the terminal of the contact suspect recorded in the electronic medical questionnaire of the confirmed examinee, and the diagnosis request text message and the electronic medical questionnaire are transmitted in the form of a push message.

Preferably, the personal medical record method related to noncontact screening treatment for a large-scale infectious disease may be provided to
provide a prescription service of transmitting at least one among a diagnosis result of the medical institution terminal, a medical prescription, and payment information to the examinee terminal in a noncontact manner.

Preferably, the personal medical record method related to noncontact screening treatment for a large-scale infectious disease may be provided to
provide a diagnosis result certification service of the examinee by matching, storing, and managing the diagnosis result of the medical institution terminal and personal identification information of the examinee, and transmitting a diagnosis result certificate for the large-scale infectious disease in response to a request of the examinee terminal.

Preferably, the personal medical record method related to noncontact screening treatment for a large-scale infectious disease may be provided to
provide a visit certification service of certificating a visit to a corresponding store in response to a request of the examinee terminal by collecting and managing information on the visit to the store acquired through a tagging operation of the examinee terminal on an entrance terminal provided at a predetermined location of the visited store.

### ADVANTAGEOUS EFFECTS

According to an embodiment, as the work of preparing an electronic medical questionnaire including a preliminary epidemiological investigation, making a diagnosis appointment, and conducting a diagnosis for a large-scale infectious disease is performed in a noncontact manner using a cloud-based open mobile platform having an open source by utilizing cloud computing, a large-scale mass infection can be prevented in advance by making a screening treatment appointment and conducting a diagnosis in a noncontact manner.

In addition, according to an embodiment, as terminal information of a contact suspect is recorded in the electronic medical questionnaire of an examinee and a push message is transmitted to the contact suspect as soon as the examinee is confirmed as being infected, large-scale mass propagation of the large-scale infectious disease can be prevented in advance.

In addition, a prescription service of transmitting a diagnosis result of the medical institution terminal, a medical prescription, and payment information may be provided to the examinee terminal in a noncontact manner, a diagnosis certification service of certificating a diagnosis result about a large-scale infectious disease may be provided on the basis of the diagnosis result of the medical institution terminal, and a visit certification service may be provided by collecting and managing information on a visit to a corresponding store acquired through a tagging operation of the examinee terminal on an entrance terminal provided at a predetermined location of the visited store.

### BRIEF DESCRIPTION OF THE DRAWINGS

Since the following drawings attached to this specification illustrate preferred embodiments of the present invention for further understanding of the technical spirit of the present invention, together with the detailed description of the present invention, the present invention should not be construed as being limited only to the matters described in the drawings.
FIG. 1 is a view showing the configuration of a noncontact screening treatment system according to an embodiment.
FIG. 2 is a view showing the detailed configuration of a management server of the system according to an embodiment.
FIG. 3 is another exemplary view showing a labeling barcode of the system in an embodiment.
FIG. 4 is a view showing the detailed configuration of a medical institution terminal of the system according to an embodiment.
FIG. 5 is an exemplary view showing the flowchart of a noncontact screening treatment system according to an embodiment.

### BEST MODE FOR CARRYING OUT THE INVENTION

Hereinafter, embodiments of the present invention will be described in more detail with reference to the drawings.

The advantages and features of the present invention and the methods for achieving them will become clear with reference to the embodiments described below together with the accompanying drawings. However, the present invention is not limited to the embodiments described below and may be implemented in various different forms, and these embodiments are provided only to make the disclosure of the present invention complete, and to fully inform those skilled in the art of the scope of the present invention, and the present invention is only defined by the scope of the claims.

The terms used in this specification will be briefly described, and the present invention will be described in detail.

Although general terms widely used in the present are selected as the terms used in the present invention as much as possible while considering the functions in the present invention, this may vary depending on the intention of those skilled in the art, precedents, emergence of new technologies, or the like. In addition, in a specific case, there are also terms arbitrarily selected by the applicant, and in this case, the meaning will be described in detail in the description of the present invention. Therefore, the terms used in the present invention should be defined based on the meaning of the terms and the overall content of the present invention, not simply based on the names of the terms.

Hereinafter, embodiments of the present invention will be described in detail so that those skilled in the art may easily practice with reference to the accompanying drawings. In addition, in order to clearly explain the present invention in the drawings, parts irrelevant to the description will be omitted.

Hereinafter, a noncontact screening treatment system of a large-scale infectious disease according to an embodiment of the present invention and a method thereof will be described in detail with reference to the accompanying drawings.

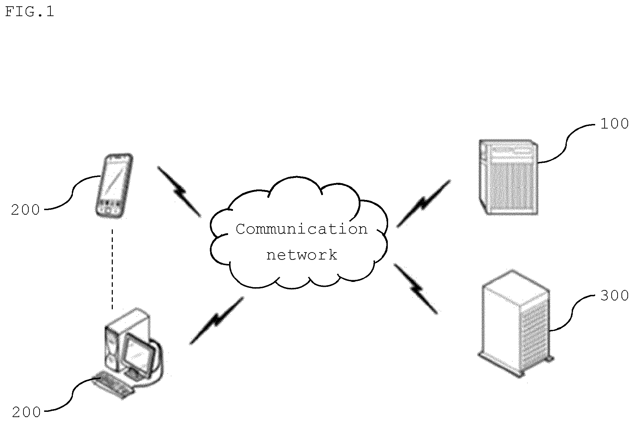

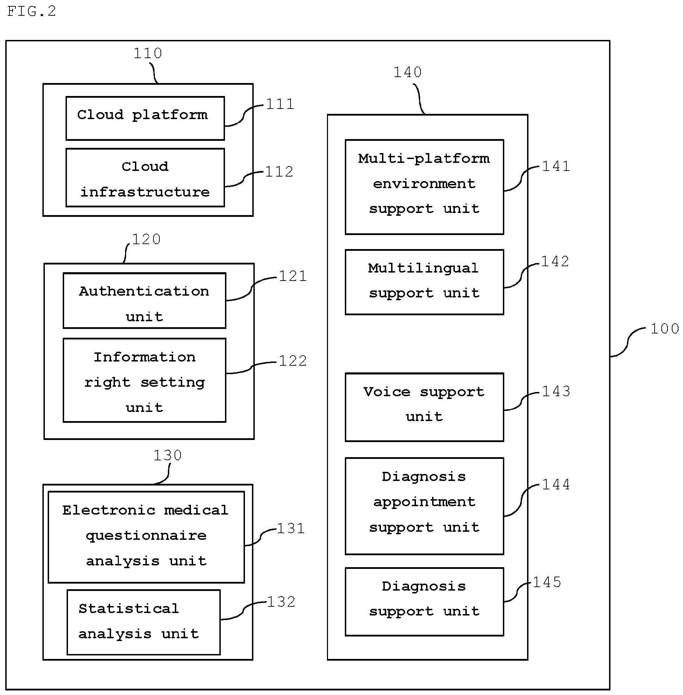

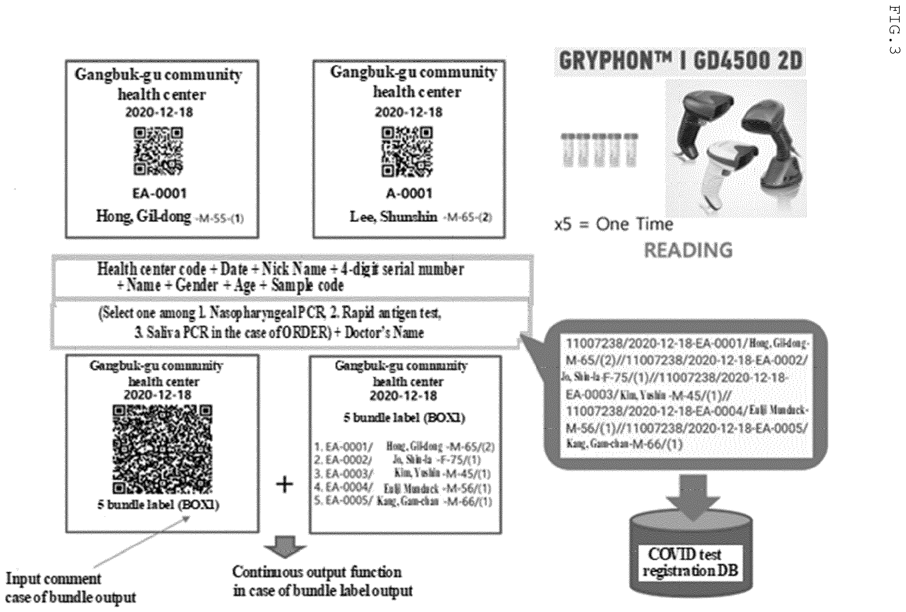

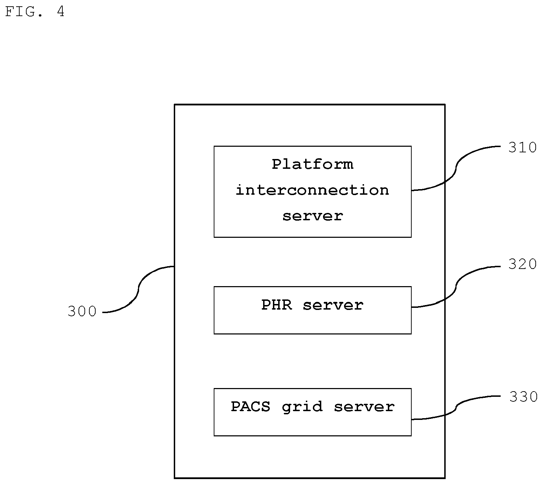

FIG. 1 is a view showing the overall configuration of a personal medical record system related to noncontact screening treatment for a large-scale infectious disease, FIG. 2 is a view showing the detailed configuration of the management server of FIG. 1, FIG. 3 is an exemplary view showing a labeling QR code generated by the diagnosis appointment support unit 144 of the management server shown in FIG. 1, and FIG. 4 is a view showing the detailed configuration of the medical institution terminal of FIG. 1. Referring to FIGS. 1 to 4, a noncontact screening treatment system of a large-scale infectious disease of an embodiment is provided to construct a diagnosis mobile platform for a large-scale infectious disease; prepare an electronic medical questionnaire including a preliminary epidemiological investigation for a large-scale infectious disease, make a diagnosis appointment, and conduct a diagnosis in a noncontact manner using the screening treatment mobile platform constructed in advance; and transmit a diagnosis request text message and the electronic medical questionnaire in the form of a push message on the basis of a diagnosis result using terminal information of a contact suspect recorded in the electronic medical questionnaire of a confirmed examinee, and therefore, the system may include at least one among a management server 100, a plurality of examinee terminals 200, and a medical institution terminal 300. For example, terminal information of a contact suspect may be a communication subscription number, and another example may be personal information such as an e-mail address or the like of the contact suspect.

Here, the management server 100 is constructed as a cloud-based open mobile platform having an open source by utilizing cloud computing to provide a mobile app for providing a prior screening treatment appointment service for an infectious disease, and provides an app or an integrated mobile app, and it can be accessed through the web and performs the functions of presiding or mediating the prior screening treatment appointment and diagnosis service for the infectious disease.

Here, the cloud computing is a computing environment in which IT-related services such as data storage, network, use of contents, and the like can be used at the same time through the management server 100 on the Internet, which is a concept that all user information can be stored in the management server 100 on the Internet and all the information can be accessed anytime and anywhere through various IT devices, and it is a computing service of borrowing computing resources such as hardware and software existing in an intangible form as much as needed, and paying for using the computing resources.

As shown in FIG. 2, the management server 100 may include at least one among a cloud service module 110, a security module 120, a user support module 130, and an integrated operation module 140.

The cloud service module 110 may construct a cloud platform 111 and a cloud infrastructure 112 to provide cloud-based services when it is connected and accessed using an app or web.

Here, the cloud-based service refers to a service of transmitting an electronic medical questionnaire received from the medical institution terminal 300 to an examinee terminal 200 connected to a corresponding app or web before visiting a screening clinic in the case of an emergency disease that may lead to a large-scale mass infection, and making a prior diagnosis appointment, as the electronic medical questionnaire prepared by the examinee terminal 200 is transmitted to the medical institution terminal 300, on the basis of the contents written in the electronic medical questionnaire prepared by the examinee. Therefore, preparing an electronic medical questionnaire and making a prior diagnosis appointment are performed in a noncontact manner on the basis of the cloud-based service, rather than visiting a corresponding screening clinic.

Here, the electronic medical questionnaire may include personal identification information such as a name, resident registration number, gender, nationality, residential address, contact information, occupation, and the like, clinical symptom information including revealed symptoms, date and time, places, use of fever reducer, underlying diseases, and smoking, revealed disease information including previous visits to medical institutions and diagnoses, and preliminary epidemiological investigation information including travel history and duration (departure dates, visited areas (countries, city names) in the last 14 days, transits before entry and details thereof (names of countries and cities, staying outside a transit airport and duration of the stay, etc.), purpose of visit, information related to companions, infection risk factors (contact with various risk factors, overlapped movement paths, etc.), entry information (date, flight number, etc.), confirmation information (quarantine information upon entry, information on contacted people, etc.), and terminal information of a contact suspect, and the like.

The information recorded in the electronic medical questionnaires is a configuration already applied to medical questionnaires provided offline for COVID-19, and although the items described in the electronic medical questionnaire are not specified in detail in this specification, it should be understood at the level of those skilled in the art.

The cloud platform 111 may be constructed on the basis of CloudFoundry to be constructed as an open type through an open source, and the cloud infrastructure 112 may be constructed on the basis of OpenStack to be constructed as an open type through an open source.

The cloud platform 111 includes a web server for providing web services based on the contents of the electronic medical questionnaire of an examinee, a framework for providing services of various languages, middleware for supporting smooth communication in a complex heterogeneous environment, a database (DB) for storing and managing various data, and an Application Programming Interface (API) gateway for supporting a mobile environment, and the cloud platform 111 supports a cloud infrastructure in association with the cloud infrastructure 112.

The cloud infrastructure 112 may include at least one among a management server control unit for managing virtual servers, such as creating and deleting virtual servers and changing metadata, a storage control unit for performing storage control, such as creating, deleting, and changing a storage, as well as linking the storages, a network control unit for performing network control, an orchestration automation unit for performing orchestration by controlling all resources including the storage, network, and the like, and managing VM groups and templates, such as creating and deleting VM groups and templates by virtual machine (VM) group, and a VM scheduling unit for managing scheduling based on the VM group 133, memory, and CPU.

The security module 120 includes an authentication unit 121 and an information right setting unit 122, and may support four authentication methods for enhancing access to the management server 100 and authentication security for the examinee or medical staff toward the management server 100, and manage and set a user's access right to data and services for each of various people of interest.

Here, the authentication unit 121 applies an open authentication (Oauth) method to provide a flexible security authentication service according to an internal situation of the management server 100, and may be constructed in a way of utilizing a resource server and an Oauth server.

That is, as a method of calling a resource web application programming interface (API) that the management server 100 has, the authentication unit 121 supports an Authorization Code Grant authentication method for trusted users, an Implicit Grant authentication method using an authentication method of a browser-based application or a mobile application widely used by public users, a Password Credentials Grant authentication method that allows users to directly access using an ID and a password, and a Client Credentials Grant authentication method that conducts authenticates using an ID and a password when an access application is a trusted user. Identity authentication can be performed using the authentication methods by inputting an authentication number that is transmitted to the subscription number of the examinee terminal 200.

For example, the information right setting unit 122 may allow the examinee to access the management server 100, and set an access right so that the examiner (medical institution) may access the diagnosis appointment service.

The integrated management module 130 may include an electronic medical questionnaire analysis unit 131 and a statistical analysis unit 132, and may analyze each item of the electronic medical questionnaire provided by the examinee terminal 200 for a prior screening treatment appointment, and receive diagnosis support of medical staff having the medical institution terminal 300 that has received an analysis result.

Accordingly, the integrated management module 130 for making a screening treatment appointment may determine an isolation stage, such as self-quarantine, visit to a screening clinic, or the like, on the basis of the contents recorded in the electronic medical questionnaire using the Clinical Decision Support System (CDSS) of the medical institution terminal 300, and introduce nearby screening clinics according to the determined isolation stage.

That is, before visiting a screening clinic for a large-scale contagious disease, an electronic medical questionnaire for a prior diagnosis appointment received from the management server 100 needs to be prepared using the examinee terminal 200 possessed by the examinee.

In addition, the electronic medical questionnaire for making a diagnosis appointment may further include terminal information of a person suspected to have been in contact with the examinee.

When it is diagnosed that a large-scale infectious disease is suspected, the electronic medical questionnaire for making a diagnosis appointment may further include appointment information such as screening clinics of a public health center with or without a copay, a public medical institution, a medical institution having previous agreements with an insurance company, or the like according to insurance review guidelines, support guidelines of the central government and local governments, and the like.

The electronic medical questionnaire analysis unit 131 performs a function of converting the electronic medical questionnaire prepared by the examinee terminal 200 into an XML-based Continuity of Care Record (CCR) document.

As the Telecommunications Technology Association (TTA) standardizes a CCR document model by standardizing the format and object definition of CCR documents, a unified general-purpose service may be provided for XML-based CCR documents.

In addition, the electronic medical questionnaire analysis unit 131 may generate big data by mining personal information or the like, in addition to the CCR documents.

Meanwhile, the statistical analysis unit 132 may grasp the overall status of patients infected with a large-scale infectious disease by analyzing the number of examinees suspected of being infected with a large-scale infectious disease, who are preparing or have completed an electronic medical questionnaire, examinees arrived at the screening clinic with an appointment among the suspected examinees, examinees currently under diagnosis, people suspected to have been in contact with a confirmed examinee, and the like, and analyze the diagnosis status of each screening clinic on the basis of the number of examinees having an appointment and examinees having been diagnosed at each screening clinic, and limit the number of diagnosis examinees by matching the number of examinees to the current status of the screening clinic. In addition, wide spread of disease may be prevented in advance by transmitting a diagnosis request text message and a generated electronic medical questionnaire to those suspected to have been in contact with a confirmed examinee in the form of a push message.

The user support module 140 includes at least one among a multi-platform environment support unit 141, a multilingual support unit 142, a voice support unit 143, a diagnosis appointment support unit 144, and a diagnosis support unit 145, and may apply web standards for an examiner to accommodate various requirements needed for diagnosing an infectious disease of an examinee, as well as access of the examinee or the examiner through an app, provide web contents accessibility for the convenience of the examinee or the examiner, support web compatibility for Internet examinees or the examiner having various access environments, support multiple languages through a multilingual conversion engine, and input voices using a voice recognition engine.

Here, the multi-platform environment support unit 141 complies with the open Internet standard recommendation (W3C) used as a standard and complies with web content accessibility guidelines, and may support web standards, web accessibility, and web compatibility by excluding an environment dependent on a specific platform and constructing to be able to serve all browsers.

On the other hand, the multilingual support unit 142 is for allowing foreign examinees to make an appointment for prior diagnosis at a screening medical institution anytime and anywhere, and is provided to support multiple languages such as English, Chinese, Japanese, and Arabic, and may be easily accessed by foreign examinees.

In addition, the voice support unit 143 allows to prepare an electronic medical questionnaire and an electronic medical questionnaire for prior diagnosis appointment by voice using a natural language processing and voice recognition engine of a chatbot, and may support preparation of an electronic medical questionnaire and a prior diagnosis appointment by voice for elderly and disabled people having difficulties in accessing the web.

The diagnosis appointment support unit 144 may assign a diagnosis appointment number to the examinee terminal 200 on the basis of the appointment items of the electronic medical questionnaire input through the examinee terminal 200, and store and manage the diagnosis appointment number in a storage, and may assign a diagnosis registration number to the examinee through a diagnosis appointment number input through an authentication input unit provided at a predetermined location of a screening clinic or a license plate number having a treatment appointment acquired through a camera.

In addition, as shown in FIG. 3, the diagnosis appointment support unit 144 generates a labeling barcode by encrypting personal identification information matching the diagnosis registration number, confirms an examinee on the basis of the personal identification information and response information the examinee displayed on the screen through identification of the generated labeling barcode, and transmits the labeling barcode to the output unit in response to a doctor's test order. Accordingly, a medical accident of changing or omitting the personal identification information and the diagnosis kit of the examinee can be prevented.

Then, the labeling barcode output from the output unit is attached to the diagnosis kit of the examinee, and the diagnosis kit attached with the labeling barcode is transmitted to the medical institution. At this point, when the labeling barcode attached to the diagnosis kit is identified, the diagnosis appointment support unit 144 may store and manage the diagnosis kit in a storage by matching the diagnosis kit and the personal identification information of the labeling barcode.

Meanwhile, after the diagnosis is completed, the diagnosis support unit 145 may perform a function of transmitting a diagnosis result of the examinee received from the medical institution terminal 300 and medical prescription and payment information generated by receiving the medical prescription and payment information based on the diagnosis result to the examinee terminal 200, and may also store the medical prescription and payment information in the storage by matching the medical prescription and payment information with the personal identification information. At this point, the diagnosis support unit 145 transmits a diagnosis request text message and the generated electronic medical questionnaire in the form of a push message using terminal information of the contact suspect recorded in the electronic medical questionnaire of the confirmed examinee.

Accordingly, the medical prescription and payment information may be transmitted to the examinee terminal 200 in a noncontact manner, and documents related to insurance claims and other examination cost claims, in addition to the medical prescription and payment information, may also be transmitted to the examinee terminal 200 as needed. Here, the medical prescription may be at least one among outpatient treatment information when a patient is discharged from or non-hospitalized in the hospital, follow-up diagnosis information, medication and treatment information, and insurance-related information.

In addition, the diagnosis support unit 145 may derive reliability of the medical prescription through a follow-up investigation in the received medical prescription and treatment results of the examinee, and reliability of the medical prescription may be applied to develop vaccines or treatment candidate materials and set as a parameter value for constructing a model for estimating disease prediction, prognosis, or the like.

Meanwhile, the examinee terminal 200 is a terminal possessed by an examinee suspected of being infected with a large-scale contagious disease, and its main object is further increasing usability of the mobile device. The examinee terminal 200 may apply a cell-based hybrid app to facilitate expansion and mounting of mobile apps, or it is preferable to construct a user environment having app compatibility by constructing a mobile device API to support two mobile platforms of iOS and Android and allowing connection to both iOS and Android.

In addition, the examinee terminal 200 is configured to support a multi-platform user environment by constructing a web user environment through Windows and MacOS to be interconnected when the management server 100 is accessed through a PC, as well as a mobile device.

The examinee terminal 200 may have a configuration including a medical image conversion interface for supporting remote medical treatment in association with a domestic or foreign medical institution, i.e., the medical institution terminal 300, and it is preferable to be configured to support smooth medical image-based remote medical treatment with domestic and foreign medical institutions by applying the Digital Imaging and Communications in Medicine (DICOM) standard protocol, which is a digital image and communication standard for medical purpose.

Meanwhile, referring to FIG. 4, the medical institution terminal 300 may include at least one among a platform interconnection server 310 for performing video medical treatment and consultation with an examinee in association with the management server 100, a PHR server 320 for sharing Personal Health Record (PHR) data of the examinee between a cooperative treatment requesting medical institution and a cooperative treatment supporting medical institution, and a Picture Archiving and Communication System (PACS) grid server 330 for sharing medical image services between the cooperative treatment requesting medical institution and the cooperative treatment supporting medical institution.

Personal Health Record (PHR) data of a diagnosis examinee may be exchanged between the cooperative treatment requesting medical institution and the cooperative treatment supporting medical institution through the PHR server 320 of the medical institution terminal 300 by way of the platform interconnection server 310, and medical images may be exchanged between the cooperative treatment requesting medical institution and the cooperative treatment supporting medical institution through the Picture Archiving and Communication System (PACS) grid server 330.

Hereinafter, the noncontact screening treatment process for large-scale contagious diseases will be described in more detail.

As the management server 100 constructs a mobile device API to support two mobile platforms of iOS and Android by applying a cell-based hybrid app, the examinee terminal 200 may access the management server 100 in an iOS and Android environment, and as the management server 100 constructs a web user environment through Windows and MacOS, the examinee terminal 200 may access the management server 100 through a PC, as well as a mobile device.

The examinee is a person suspected to be exposed to a large-scale infectious disease, and access to the management server 100 may be limited by security authentication and information right setting using the security module 120 when the examinee accesses the management server 100.

Accordingly, the management server 100 has a cloud-based mobile platform constructed therein and performs a function of presiding and mediating at least one among the services of preparing an electronic medical questionnaire, making a diagnosis appointment, and conducting a diagnosis for a large-scale infectious disease.

In addition, the examinee terminal 200 operates in association with the management server and is equipped with an app for providing services of preparing an electronic medical questionnaire, making a diagnosis appointment, and conducting a diagnosis for a large-scale infectious disease, and as the app is executed, it may be provided with a noncontact electronic medical questionnaire preparation service of downloading an electronic medical questionnaire by way of the management server 100, filling out corresponding items, and then transmitting the electronic medical questionnaire to the management server 200 by pressing an input confirmation key.

That is, the management server 100 transmits an electronic medical questionnaire input window including a plurality of consent forms, personal identification information of an examinee, clinical symptoms, and a preliminary epidemiological investigation to the examinee terminal 200, and the examinee terminal 200 fills out the items of the received electronic medical questionnaire and transmits the prepared electronic medical questionnaire to the management server 100 by pressing the input confirmation key, and accordingly, the management server 100 transmits the received electronic medical questionnaire to the medical institution terminal.

Here, the consent form of the electronic medical questionnaire may include at least one among consent to collection of personal health record (PHR), consent to sharing of information among participating medical institutions, consent to sharing of personal information for research purposes, and consent to omitting input of personal identification information in the case of an anonymous diagnosis.

The electronic medical questionnaire according to symptoms of a large-scale contagious disease is prepared through standard guidelines for large-scale contagious diseases, and is already applied to large-scale contagious diseases such as COVID-19 or the like, and therefore, although it is not specified in detail in this specification, it should be understood at the level of those skilled in the art.

In addition, the management server 100 may add an item requesting an anonymous test to the personal identification information input window to prevent exposure of personal identification information, and transmit the personal identification information input window to the examinee terminal 200, and accordingly, the management server 100 performs identity authentication using the communication subscription number for receiving a notification of a test result of the examinee terminal 200 when the anonymous test item is selected, and then when the identity authentication is successful, the management server 100 transmits an electronic medical questionnaire input window omitting input of the personal identification information item to the examinee terminal 200, and therefore, the electronic medical questionnaire may be prepared in a noncontact manner.

The examinee terminal 200 transmits the prepared electronic medical questionnaire to the management server 100 when the input confirmation key is pressed after preparation of the electronic medical questionnaire is completed, and the management server 100 matches, stores, and manages the electronic medical questionnaire in the storage the electronic medical questionnaire and the personal identification information.

In addition, the management server 100 transmits the received electronic medical questionnaire to the medical institution terminal 300, and the medical institution terminal 300 converts the electronic medical questionnaire collected by a plurality of examinee terminals 200 into XML-based CCR documents, and stores and manages electronic medical questionnaires of a unified format in the storage by matching the electronic medical questionnaire and the personal information.

Then, the medical institution terminal 300 determines an isolation stage on the basis of the unified electronic medical questionnaire, and transmits the determined isolation stage to the examinee terminal 200 via the management server 100.

Here, the isolation stage for the large-scale contagious diseases includes an observation stage of requiring follow-up observation for a predetermined period of time when the symptom of a large-scale contagious disease is mild as a result of the analysis, a visit request stage of requiring a prompt visit to a screening clinic when the symptom of a large-scale contagious disease is severe as a result of the analysis, and a mandatory visit stage of switching to a visit stage when the symptom is more severe as a result of confirming the symptom after the observation stage.

The isolation stage according to the symptom of a large-scale contagious disease is a stage already applied to COVID-19 or the like, and although it is not specified in detail in this specification, it should be understood at the level of those skilled in the art.

Meanwhile, when the isolation stage is a visit request stage and a mandatory visit stage requiring a visit to a screening clinic, the management server 100 transmits an appointment information input window for providing a diagnosis appointment service to the examinee terminal 200.

That is, the management server 100 maps a selection window that displays a plurality of screening clinics available for making an appointment to map data, and transmits the window to the examinee terminal 200. Here, the plurality of selected screening clinics is one of screening clinics with or without a copay among the plurality of screening clinics on the basis of the constraints of the examinee set by the government organization.

Accordingly, the management server 100 transmits an appointment information input window including an available diagnosis appointment date of a screening clinic selected among the screening clinics displayed on the examinee terminal 200, a time slot, a diagnosis method including face-to-face diagnosis or drive-through diagnosis, and a license plate number when the drive-through diagnosis is selected to the examinee terminal 200.

When the examinee terminal 200 inputs the items of appointment information and the input confirmation key is pressed, the management server 100 generates a diagnosis appointment number including the appointment status of the selected screening clinic on the basis of the received appointment information, and transmits the diagnosis appointment number to the examinee terminal 200.

Thereafter, the management server 100 performs registration of screening treatment for a large-scale infectious disease in a noncontact manner when the examinee arrives at the selected screening clinic, and generates a diagnosis registration number matching the diagnosis appointment number at the time of arrival authentication on the basis of the diagnosis appointment number of the authentication input unit provided at a predetermined location of the selected screening clinic or a license plate number acquired through a camera.

At this point, the management server 100 may provide voice support through the voice support unit 143 for disabled or elderly people, as well as language support through the multilingual support unit 142 when the examinee is a foreigner.

The management server 100 may perform an arrival authentication of the examinee on the basis of the diagnosis appointment number input through the authentication input unit provided at a predetermined location of the screening clinic or a license plate number acquired through a camera, and assign a diagnosis registration number to the examinee when the arrival authentication is successful.

At this point, the diagnosis registration number is assigned to the examinee terminal 200 according to the order of arrival authentication of the examinee terminal 200, and the assigned diagnosis registration number is stored and managed in the storage to be matched to the electronic medical questionnaire of the examinee.

In addition, the management server 100 may provide the medical staff through the medical institution terminal 300 of the images of arrival at the screening clinic and the screening treatment process of the examinee transmitted through the examinee terminal 200.

The management server 100 may generate a labeling barcode in which personal identification information matching the generated diagnosis registration number is encrypted and stored, attach the labeling barcode to the diagnosis kit of the examinee after confirming identity of the generated labeling barcode, and transmit the diagnosis kit to the medical institution terminal 300. At this point, the labeling barcode may be matched to the personal identification information and stored and managed in the storage. Accordingly, medical accidents such as switching the examinee and the test sample can be prevented.

Then, the medical institution terminal 300 generates a diagnosis result, a medical prescription, and payment information for a large-scale infectious disease according to a result of analyzing the test sample of the diagnosis kit, PHR data of the diagnosis examinee of the PHR server 320 connected through the platform interconnection server 310, and medical image data of the PACS grid server 330, and transmits the generated diagnosis result, medical prescription, and payment information to the management server 100.

When the examinee interfaced with the management server 100 selects a domestic or foreign medical institution and medical staff provided through a screen displayed on the management server 100 and requests video inquiry, the video inquiry and its response may be mediated through an interface with a corresponding medical institution terminal 300.

The management server 100 may store and manage diagnosis results of the medical institution terminal 300, and perform certification on the diagnosis result by transmitting a diagnosis result for the large-scale infectious disease to the examinee terminal 200 in response to the request of the examinee terminal 200.

In addition, the management server 100 transmits a diagnosis request text message and the generated electronic medical questionnaire using terminal information of a contact suspect on the basis of the electronic medical questionnaire of the confirmed examinee in the form of a push message.

In addition, the management server 100 may provide a visit certification service by acquiring identification information recorded in an entrance terminal provided at a predetermined location of a visited store by a tagging operation of the examinee terminal 200 on an entrance terminal, storing and managing the acquired information on the visit to the store, and transmitting the visit information to the examinee terminal 200.

Accordingly, in an embodiment, as the work of preparing an electronic medical questionnaire, making a diagnosis appointment, and conducting a diagnosis is carried out in a noncontact manner in real-time for a large-scale infectious disease, mass infection that may occur when existing paper medical questionnaires are prepared can be prevented, and corresponding organizations such as health authorities or the like may manage information and quickly grasp the status of large-scale infection in real-time.

In addition, in an embodiment, as a multilingual support service for foreigners and a voice support service for disabled and elderly people are provided, it is possible to easily prepare an electronic medical questionnaire and make a screening clinic appointment.

In an embodiment, as the diagnosis appointment number or the barcode generated when a prior diagnosis appointment is made on the basis of a mobile technique is transmitted to the examinee terminal by interconnection among the management server, the examinee terminal, and the medical institution terminal, and the diagnosis registration number is assigned to the examinee terminal when arrival authentication is performed at the screening clinic by the operation of a camera that captures the barcode or the license plate number of the examinee registered when the diagnosis appointment is made or by the operation of pressing the authentication input unit, registration of a diagnosis can be made at a screening clinic in a noncontact manner in real-time, and a large-scale infectious disease can be diagnosed in an early stage.

In an embodiment, in the case of overseas arrivals, since electronic medical questionnaires prepared based on an app or web are stored and managed on the basis of cloud of the management server, duplicated preparation of electronic medical questionnaires can be prevented.

In an embodiment, since visits to a store where there is a risk of infection with a large-scale infectious disease can be confirmed through a tagging operation on an entrance terminal provided at a predetermined location in the visited store, and anonymity of the personal information of an examinee, which can be exposed in the process of tracking the movement of the examinee for a large-scale infectious disease, is guaranteed, privacy of the examinee can be protected, and diagnosis of those who do not or passively participate in the diagnose of a large-scale infectious disease can be conducted.

In addition, according to an embodiment, as terminal information of a contact suspect is recorded in the electronic medical questionnaire of an examinee and a push message is transmitted to the contact suspect as soon as the examinee is confirmed as being infected, large-scale mass propagation of the large-scale infectious disease can be prevented in advance.

In an embodiment, since a result of diagnosis for a large-scale infectious disease, and medical prescription and payment information for the diagnosis result are transmitted to the examinee terminal via the management server, the diagnosis result and medical prescription can be output on the examinee terminal in the form of a text message or 2D barcode in a noncontact manner in real-time, and customs clearance authentication can be performed at workplaces, quarantine authorities, administrative authorities in charge of health and public security, local government health authorities, overseas immigration offices, international airports, international quarantine offices, and the like using the examinee terminal.

In addition, in an embodiment, medical accidents such as switching an examinee and a test sample of a diagnosis kit can be prevented through the labeling barcode in which the personal identification information of the examinee of the electronic medical questionnaire prepared before visiting the screening clinic is encrypted and recorded.

In an embodiment, since standard guidelines for a large-scale infectious disease are transmitted to a plurality of screening clinics in real-time through web access of the management server 100, efficiency of performing a work at the plurality of screening clinics can be maximized.

In addition, in an embodiment, since diagnosis results and medical prescriptions are managed in a noncontact manner in real-time, the management server 100 may perform statistical analysis of a large-scale infectious disease, rapidly develop treatment candidate materials, vaccines, and the like, and quickly perform procedures such as drug prescription, treatment, and insurance claims.

Accordingly, in an embodiment, a cloud-based open mobile platform can be constructed, and cloud-based diagnosis appointments and diagnosis services for a large-scale infectious disease, which can enhance utilization of mobile devices, can be provided, and in addition to improving the accessibility and convenience of a web for making a diagnosis appointment and conducting a diagnosis for a large-scale infectious disease using a mobile device, domestic as well as overseas competitiveness can be secured by attracting foreign patients.

In another aspect of an embodiment, a noncontact screening treatment method performed by a management server of a noncontact screening treatment device for a large-scale infectious disease includes the steps of: mounting an app of the management server for providing an electronic medical questionnaire preparation service for the large-scale infectious disease on an examinee terminal, and executing the mounted app; transmitting, by the management server, an electronic medical questionnaire input window including a plurality of consent forms, personal identification information of an examinee, clinical symptoms, terminal information of a contact suspect, and a preliminary epidemiological investigation to the examinee terminal; and transmitting, by the management server after preparing the electronic medical questionnaire, the received electronic medical questionnaire to a medical institution terminal by pressing an input confirmation key of the examinee terminal, and the method further includes a configuration for providing, by the management server, a diagnosis appointment service for the examinee according to an isolation stage received from the medical institution terminal, and conducting diagnosis of a large-scale infectious disease in a noncontact manner when the examinee arrives at a selected screening clinic, and since each step of the noncontact screening treatment method is a function performed by the management server 100, the examinee terminal 200, and the medical institution terminal 300, detailed description thereof is omitted.

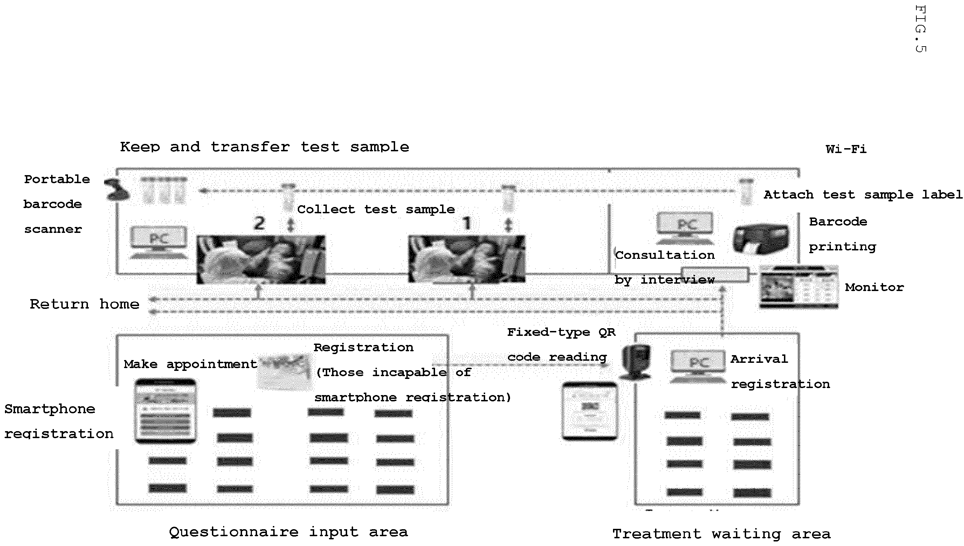

FIG. 5 is an exemplary view showing the layout and medical treatment flow of the management server shown in FIG. 1. Referring to FIG. 4, it can be known that as the work of preparing an electronic medical questionnaire, making a diagnosis appointment, and conducting a diagnosis is carried out in a noncontact manner in real-time for a large-scale infectious disease, mass infection that may occur when existing paper medical questionnaires are prepared can be prevented, and corresponding organizations such as health authorities or the like may manage information and quickly grasp the status of large-scale infection in real-time.

Although the present invention has been described above in detail through the representative embodiments, those skilled in the art will understand that various modifications are possible to the above-described embodiments without departing from the scope of the present invention. Therefore, the scope of the present invention should not be defined to be limited to the embodiments described above, and should be defined by all changed or modified forms derived from the claims and equivalent concepts, as well as the claims described below.

## Claims

1. A personal medical record system related to noncontact screening treatment for a large-scale infectious disease, the system comprising:
a management server having a cloud-based mobile platform constructed therein to preside and mediate preparation of an electronic medical questionnaire including a preliminary epidemiological investigation for the large-scale infectious disease at an appointment stage; and
an examinee terminal operating in association with the management server and equipped with an app for providing an electronic medical questionnaire preparation service including a preliminary epidemiological investigation for the large-scale infectious disease at the appointment stage to download an electronic medical questionnaire input window by executing the app and transmit the prepared electronic medical questionnaire to the management server by pressing an input confirmation key.

2. The system according to claim 1, wherein the management server performs the electronic medical questionnaire preparation service in a noncontact manner by transmitting an electronic medical questionnaire, which contains a plurality of forms of consent to information sharing and inquiries about personal identification information of the examinee, clinical symptoms, terminal information of a contact suspect, and the preliminary epidemiological investigation, to the subject terminal, and transmitting the received prepared electronic medical questionnaire to a medical institution terminal by pressing the input confirmation key of the examinee terminal.

3. The system according to claim 2, wherein the consent form of the electronic medical questionnaire includes at least one among consent to collection of personal health record (PHR), consent to sharing of information among participating medical institutions, consent to sharing of personal information for research purposes, and consent to omitting input of personal identification information in the case of an anonymous diagnosis, and the preliminary epidemiological investigation is included in the appointment stage.

4. The system according to claim 2, wherein as an anonymous test item for preventing exposure of the personal identification information is added to the electronic medical questionnaire in the electronic medical questionnaire input window, the management server is provided to perform identity authentication using a communication subscription number of the examinee for receiving a notification of a test result, and prepare an electronic medical questionnaire omitting input of a personal identification information item when the identity authentication is successful.

5. The system according to claim 2 or 4, wherein the management server is provided to provide a diagnosis appointment service for the examinee according to an isolation stage received from the medical institution terminal, and the management server is provided to provide the diagnosis appointment service in a noncontact manner by mapping a plurality of screening clinics available for making an appointment to map data on the basis of an isolation measure of the medical institution terminal set based on the clinical symptoms and preliminary epidemiological investigation of the electronic medical questionnaire, and transmitting the screening clinics to the examinee terminal, transmitting an input window of diagnosis appointment information, including an appointment date of a screening clinic selected among displayed screening clinics, a time slot, a diagnosis method of noncontact diagnosis or drive-through diagnosis, and a license plate number when the drive-through diagnosis is selected, to the examinee terminal, and generating a diagnosis appointment number including appointment status of the selected screening clinic on the basis of the received appointment information when corresponding items of the diagnosis appointment information are input and the input confirmation key is pressed and transmitting the diagnosis appointment number to the examinee terminal.

6. The system according to claim 5, wherein the plurality of selected screening clinics selected by the management server is one of a screening clinic with a copay or a screening clinic without a copay among the plurality of screening clinics according to contents of the electronic medical questionnaire filled out in advance.

7. The system according to claim 5, wherein the management server is provided to provide a diagnostic test service for a large-scale infectious disease when the examinee arrives at the selected screening clinic, and the management server is provided to perform an arrival authentication of the examinee at the selected screening clinic by inputting the diagnosis appointment number through an authentication input unit provided at a predetermined location of the selected screening clinic or based on a barcode or license plate number acquired through a camera, generate a diagnosis registration number matching the diagnosis appointment number when the authentication is successful, generate as needed a labeling barcode, in which personal identification information matching the generated diagnosis registration number is encrypted and stored, after a doctor orders a test, and perform identity authentication for the generated labeling barcode, attach the labeling barcode to a diagnosis kit of the examinee, and transmit the diagnosis kit to the medical institution terminal when the authentication is successful.

8. The system according to claim 7, wherein the management server is provided to provide a prescription service of transmitting at least one among a diagnosis result of the medical institution terminal, a medical prescription, and payment information to at least one among the examinee terminal, a drug store, a courier server, and a payment server in a noncontact manner.

9. The system according to claim 7, wherein the management server is provided to provide a diagnosis result certification service of certificating a diagnosis result about a large-scale infectious disease by storing and managing diagnosis results of the medical institution terminal by personal identification information and transmitting the diagnosis result to the examinee terminal in a noncontact manner in real-time.

10. The system according to claim 7, wherein the management server is provided to transmit a diagnosis request text message and an electronic medical questionnaire generated based on the diagnosis result to the terminal of the contact suspect recorded in the electronic medical questionnaire of a confirmed examinee, and the diagnosis request text message and the electronic medical questionnaire are transmitted in the form of a push message.

11. The system according to claim 7, wherein the management server is provided to provide a visit certification service by collecting and managing information on a visit to a corresponding store acquired through a tagging operation of the examinee terminal on an entrance terminal provided at a predetermined location of the visited store, and transmitting information on the visit to the corresponding store to the examinee terminal in response to a request of the examinee terminal.

12. A personal medical record method related to noncontact screening treatment for a large-scale infectious disease performed by a noncontact screening treatment appointment system including a management server having a cloud-based mobile platform and an examinee terminal connected based on a web, the method comprising the steps of:
mounting an app (including programs running on a PC or mobile web browser) of the management server for providing an electronic medical questionnaire preparation service including a preliminary epidemiological investigation for the large-scale infectious disease on the examinee terminal, and executing the mounted app;
transmitting, by the management server, an electronic medical questionnaire input window, containing a plurality of forms of consent to information sharing and inquiries about personal identification information of an examinee, clinical symptoms, terminal information of a contact suspect, and the preliminary epidemiological investigation, to the examinee terminal; and
transmitting, by the management server after preparing the electronic medical questionnaire, the received electronic medical questionnaire to the medical institution terminal by pressing an input confirmation key of the examinee terminal.

13. The method according to claim 12, providing a diagnosis appointment service for the examinee according to an isolation stage received from the medical institution terminal, by the management server, and further comprising the steps of:
mapping a plurality of screening clinics available for making an appointment to map data on the basis of an isolation stage set based on the clinical symptoms and preliminary epidemiological investigation received from the medical institution terminal, and transmitting the screening clinics to the examinee terminal, by the management server;
transmitting an input window of appointment information, which includes an available date of a screening clinic selected among displayed screening clinics, a time slot, a diagnosis method of noncontact diagnosis or drive-through diagnosis, and a license plate number when the drive-through diagnosis is selected, to the examinee terminal; and
generating a diagnosis appointment number including appointment status of the selected screening clinic on the basis of the received appointment information when each item of the appointment information is input and the input confirmation key of the examinee terminal is pressed and transmitting the diagnosis appointment number to the examinee terminal.

14. The method according to claim 13, further comprising the steps of:
conducting diagnosis of a large-scale infectious disease in a noncontact manner when the examinee arrives at the selected screening clinic, by the management server, and generating a diagnosis registration number matching the diagnosis appointment number at the time of arrival authentication of the examinee on the basis of input of the diagnosis appointment number of the authentication input unit provided at a predetermined location of the selected screening clinic, or a barcode or a license plate number acquired through a camera;
generating a labeling barcode, in which personal identification information matching the previously generated diagnosis registration number is encrypted and stored, after a doctor orders diagnosis; and
attaching the labeling barcode to a diagnosis kit of the examinee, and transmitting the diagnosis kit to the medical institution terminal when identity authentication for the generated labeling barcode is successful.

15. The method according to claim 13, wherein the management server transmits a diagnosis request text message and an electronic medical questionnaire, which is generated when the examinee is confirmed to be infected as a result of the diagnosis, to the terminal of the contact suspect recorded in the electronic medical questionnaire of the confirmed examinee, and the diagnosis request text message and the electronic medical questionnaire are transmitted in the form of a push message.

16. The method according to claim 13, further comprising the step of providing a prescription service of transmitting at least one among a diagnosis result of the medical institution terminal, a medical prescription, and payment information to the examinee terminal in a noncontact manner.

17. The method according to claim 14, further comprising the step of providing a diagnosis result certification service of the examinee by matching, storing, and managing the diagnosis result of the medical institution terminal and personal identification information of the examinee, and transmitting a diagnosis result certificate for the large-scale infectious disease in response to a request of the examinee terminal.

18. The method according to claim 17, further comprising the step of providing a visit certification service of certificating a visit to a corresponding store in response to a request of the examinee terminal by collecting and managing information on the visit to the store acquired through a tagging operation of the examinee terminal on an entrance terminal provided at a predetermined location of the visited store.

19. A computer-readable recording medium on which a program for executing the personal medical record method related to noncontact screening treatment for a large-scale infectious disease according to any one of claims 12 to 18 is recorded.
